# EUROPEAN PATENT SPECIFICATION

(11) **EP 2 533 777 B1**
(45) Date of publication and mention of the grant of the patent: **06.07.2016**
(21) Application number: 11706964.1
(22) Date of filing: 28.01.2011
(51) Int. Cl.: A61K 31/381, A61K 31/46, A61K 31/575, A61K 9/00, A61P 11/00, A61P 11/06

(54) **PHARMACEUTICAL COMPOSITIONS COMPRISING FLUTICASONE, TIOTROPIUM AND SODIUM CROMOGLYCATE**
PHARMAZEUTISCHE ZUSAMMENSETZUNGEN MIT FLUTICASON, TIOTROPIUM UND NATRIUMCROMOGLYCAT
COMPOSITIONS PHARMACEUTIQUES CONTENANT DE LA FLUTICASONE, DU TIOTROPIUM ET DU CROMOGLYCATE DE SODIUM

(30) Priority: 02.02.2010 TR 201000733
(43) Date of publication of application: 19.12.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2011/000024
(87) International publication number: WO 2011/096909

(56) References cited:
- EP-A1- 0 418 716
- WO-A1-01/78739
- WO-A1-2004/110404
- WO-A1-2005/053648
- US-A- 4 335 121
- US-A1- 2002 183 292
- CAZZOLA ET AL: "A pilot study to assess the effects of combining fluticasone propionate/salmeterol and tiotropium on the airflow obstruction of patients with severe-to-very severe COPD", PULMORNARY PHARMACOLOGY & THERAPEUTICS, ACADEMIC PRESS, GB, vol. 20, no. 5, 20 July 2007 (2007-07-20), pages 556-561, XP022162533, ISSN: 1094-5539, DOI: DOI:10.1016/J.PUPT.2006.06.001

## Description

The present invention is related to a pharmaceutical composition containing fluticasone, tiotropium, cromoglicic acid and/or pharmaceutically acceptable derivatives thereof and the use of this composition in the treatment of respiratory diseases particularly in asthma, allergic rhinitis and chronic obstructive pulmonary diseases (COPD).

Airways, in other words bronchia, are the channels which function to distribute the inhaled air into the lung tissues. In the case of respiratory diseases such as asthma or chronic obstructive pulmonary disease (COPD), stimulants such as allergen, infection, good and bad smell, smoke, genetic factors and exercise cause contractions in the airway muscles (bronchoconstruction) and/or excessive secretion in glands and results in contractions in the airway; hence, respiration gets more difficult as the inhaled air cannot be exhaled.

Corticosteroids are the agents used in respiratory diseases, primarily in chronic obstructive pulmonary disease (COPD). Fluticasone, which belongs to corticosteroids group, is a strong anti-flammatuar agent that was described in patent number US 4335121 for the first time. Fluticasone represses the inflammation by preventing the inflamatuar cells to accumulate in the respiratory track. It prevents mucus aggregation by reducing the mucus secretion and provides to open the respiratory track. There is no bronchodilatory effect of the molecules in this group. For this reason, researchers provided broncodilation while preventing the formation of the inflammation by using the molecules having bronchodilator effects with corticosteroids. In general, it is possible to classify the drugs having bronchodilator effects as beta-agrenergic receptor agonists, forfodiesterez inhibitors, and anticholinergic (antimuscarinic) medicaments.

Anticholinergic medicaments present bronchodilator effects through 3 different mechanisms; (1) removing the parasympathetic tone on the bronchi, (2) inhibiting vagovagal reflexes which play a role in the formation of bronchospasm, (3) preventing the stimulation of mast cells that release molecules having bronchoconstrictor effects.

Tiotropium, which is an anticholinergic agent, was first described in the patent numbered EP0418716. Tiotropium and especially its bromide salt are indicated in the treatment of bronchial asthma and related diseases.

Salts of cromoglicic acid are used in the treatment of bronchospasm in pharmacology. Owing to this property, it is used in the treatment of asthma, allergic rhinitis, allergic and vernal conjunctivitis diseases.

Sodium cromoglycate, which was described in the patent numbered US 3419578 for the first time, is an indicated molecule in the treatment of asthma and it is contained by the products which are marketed by the trademarks of "Intal" and "Cromohexal".

The use of combination drugs in the treatment of respiratory diseases such as asthma and COPD is very effective particularly in decreasing asthma attacks. It is also possible that the severity or occurrence possibility of side effects that arise from the abovementioned active agents decreases as the active agents that are used in combinations are more effective at lower doses compared to the active substances used alone for the treatment of respiratory diseases such as asthma and COPD.

In this context the document WO01/78739 is relevant. However, decreasing the side effects that arise from the active agents is not sufficient to provide effective treatment for respiratory diseases. The medicaments used in the formulations should be selected in a way to give the best combination and furthermore, they should be in the most stable form. Moreover, the compositions comprising them should be formulated in such a way that the composition is stable and also it reaches to the target area in the most efficient way.

The inventor has surprisingly found that unexpected therapeutic benefits are obtained through the use of the combination comprising tiotropium bromide with water content less than or equal to 2.5%, fluticasone propionate and sodium cromoglycate together for simultaneous or sequential administration in the prevention or treatment of respiratory diseases.

It was seen that a combination comprising tiotropium bromide with water content less than or equal to 2.5%, fluticasone propionate and sodium cromoglycate provides the most stable and therapeutically beneficial combination for simultaneous or sequential administration in the prevention or treatment of respiratory diseases. Preferably, a composition comprising tiotropium bromide with water content less than or equal to 2.5%, fluticasone propionate and sodium cromoglycate is administered simultaneously.

In another aspect, said composition comprises tiotropium bromide with water content less than or equal to 2.5%, fluticasone propionate, sodium cromoglycate and lactose.

In another aspect, tiotropium bromide with water content less than or equal to 2.5%, fluticasone propionate, sodium cromoglycate are preferably combined in a single dosage form.

Furthermore, the amount of active agents used in the composition is carefully adjusted in order to prevent the side effects that might arise from these agents and it was seen that minimum side effects are observed when the ratio of tiotropium bromide with water content less than or equal to 2.5%: fluticasone propionate to sodium cromoglycate present in the composition is in the range of 1:2:40 to 1:50:2500 by weight. Furthermore, it was seen that in the formulation that is constituted with active agents which have a ratio in the range of 1:2:40 to 1:50:2500, the adhesive force between the particles is less and hence the amount of inhaled particles and efficacy of the formulation increases.

Accordingly; the present invention provides a combined drug preparation comprising tiotropium bromide with water content less than or equal to 2.5%, fluticasone propionate and sodium cromoglycate in amounts in the range of 1:2:40 to 1:50:2500 for simultaneous or sequential administration in the prevention or treatment of respiratory diseases such as asthma and chronic obstructive pulmonary diseases (COPD).

According to another aspect, the present invention provides an effective inhalation of the drug comprising tiotropium bromide with water content less than or equal to 2.5%, fluticasone propionate and sodium cromoglycate.

The drug pertaining to the present invention is preferred to be administered by the inhalation route as it a) has a more rapid onset of action compared to the administration via oral or parenteral routes b) enables the use at lower doses c) minimizes the side effects.

According to another aspect, the present invention provides simultaneous or sequential inhalation of the drug comprising tiotropium bromide with water content less than or equal to 2.5%, fluticasone propionate and sodium cromoglycate by the inhalation route.

According to another aspect, the present invention provides the transmission of the drug comprising tiotropium bromide with water content less than or equal to 2.5%, fluticasone propionate and sodium cromoglycate via single or multi dose inhalers.

The drug comprising tiotropium bromide with water content less than or equal to 2.5%, fluticasone propionate and sodium cromoglycate can be in dry powder form; they can be formulated with propellant gases to give aerosol formulations or they can be formulated with solvents to give nebulizer formulations. The inventors have found that the best way to transfer the medicament comprising tiotropium bromide with water content less than or equal to 2.5%, fluticasone propionate and sodium cromoglycate is using this medicament combination in dry powder form.

In this way, the components maintain their stability and furthermore, the medicament is in a stable form and is easily used by the patients.

In order to ensure effective absorption of the active agents into the lung tissue, the particle size of the agents should be adjusted. Although large particle size provides ease in manufacturing the dry powder, it may accumulate in throat and lead to insufficient intake of the medicament. Very fine particles, on the other hand, may reach the lungs. However, they might not have a good flow property which causes problems in providing dose accuracy in turn. To prevent these problems, the active agents should have an optimum average particle size. The inventors have found that tiotropium bromide with water content less than or equal to 2.5%, fluticasone propionate and sodium cromoglycate having a mean particle size not less than 1 µm reaches the lungs effectively and no problems related to flow properties of the dry powder are observed. Particularly, the inventors have found that when the mean particle size of tiotropium bromide with water content less than or equal to 2.5% and fluticasone propionate is in the range of 1.5 to 4.5 µm, and the mean particle size of sodium cromoglycate is in the range of 1 to 300 µm, the drug comprising the combination of said active agents reaches the lungs effectively and also no problems related to flow properties of the dry powder are observed.

In one aspect, the present invention provides a medicament composition comprising tiotropium bromide with water content less than or equal to 2.5%, fluticasone propionate and sodium cromoglycate in dry powder form wherein the mean particle size of tiotropium bromide with water content less than or equal to 2.5%, fluticasone propionate and sodium cromoglycate is not less than 1 µm and wherein said active agents are present in the composition with the ratio of 1:2:40 to 1:50:2500 respectively and said composition can be simultaneously or sequentially administered in the prevention or treatment of respiratory diseases.

The term "mean particle size" refers to particles wherein the particle size of 50% of the total number of particles is less than the average particle size.

According to the present invention, the drug comprising tiotropium bromide with water content less than or equal to 2.5%, fluticasone propionate and sodium cromoglycate may also contain effective amounts of excipients and/or additional agents apart from active agents.

According to the present invention, the dry powder formulation comprising tiotropium bromide with water content less than or equal to 2.5%, fluticasone propionate and sodium cromoglycate is transmitted to the patient in dry powder form. Said dry powder formulations also contain some physiologically acceptable excipients along with the active agent. These excipients can be monosaccharides (glucose, etc.), disaccharides (lactose, saccharose, maltose, etc.), oligosaccharides and polysaccharide (dextran, etc.), polyalcohols (sorbitol, mannitol, xylitol, etc.), salts (sodium chloride, calcium carbonate, etc.) or a mixture thereof.

The inventors have found that in compositions according to present invention, in other words in compositions comprising tiotropium bromide with water content less than or equal to 2.5%, fluticasone propionate and sodium cromoglycate in dry powder form wherein the mean particle size of tiotropium bromide with water content less than or equal to 2.5%, fluticasone propionate and sodium cromoglycate is not less than 1 µm and wherein said active agents are present in the composition with the ratio of 1:2:40 to 1:50:2500 respectively, using lactose as the one and only carrier provides optimum homogeneity and flow properties to the dry powder and this way dose accuracy is maintained.

It was also seen that the mean particle size of the carrier plays an important role in delivery of the medicament to the target area, i.e. lungs, effectively in the compositions pertaining to the present invention. It was found that when lactose having a mean particle size less than or equal to 100µm is used in compositions comprising tiotropium bromide with water content less than or equal to 2.5%, fluticasone propionate and sodium cromoglycate in dry powder form wherein the mean particle size of tiotropium bromide with water content less than or equal to 2.5%, fluticasone propionate and sodium cromoglycate is not less than 1 µm and wherein said active agents are present in the composition with the ratio of 1:2:40 to 1:50:2500 respectively, the adhesive forces present between the lactose particles and the active agents having a mean particle size not less than 1 µm are minimized and thus, an effective inhalation of the active agents takes place.

In another aspect, the present invention provides a composition comprising tiotropium bromide with water content less than or equal to 2.5%, fluticasone propionate and sodium cromoglycate in dry powder form wherein;
- the mean particle size of tiotropium bromide with water content less than or equal to 2.5%, fluticasone propionate and sodium cromoglycate is not less than 1 µm and
- said active agents are present in the composition with the ratio of 1:2:40 to 1:50:2500 respectively and
- lactose which has a mean particle size less than 100 µm is used as carrier.

Lactose which has a mean particle size less than 100 µm is preferably used as a mixture of particles having two different mean particle sizes. Accordingly, lactose which has a mean particle size less than 100 µm can be present as a mixture of particles having a mean particle size less than 10 µm (finer) and particles having a mean particle size in the range of 10 µm to 100 µm (coarser). The inventors have observed that when lactose which has two different mean particle sizes is used, the adhesive force between the active agents and lactose is even less.

The weight ratio of lactose particles having a mean particle size less than 10 µm (finer) to lactose particles having a mean particle size in the range of 10 µm to 100 µm (coarser) is in the range of 1:1 to 1:25, preferably in the range of 1:1 to 1:10, more preferably in the range of 1:1.5 to 1: 5.

According to the present invention, the amount of pharmaceutically acceptable carrier is preferably in the range of 0-50 mg.

According to another aspect, the present invention provides a method to transmit the drug combination comprising tiotropium, fluticasone and cromoglicic acid and/or pharmaceutically acceptable derivatives thereof via a dry powder inhaler in which the drug is stored in peelable blister packs, capsules or a reservoir for use in the treatment of patients suffering from respiratory diseases.

In the inhalation devices which are designed to transmit dry powder drugs, an effective amount of the dry powder drug is prepared for inhalation when the device is actuated.

In order to prepare the dry powder formulation stored in capsules, the additional components in the device provides the capsule to open or be pierced when the device is actuated and the dry powder formulation is prepared for inhalation. After the inhalation is completed, the empty capsule is removed from the device and a new capsule is placed immediately before the following inhalation takes place.

According to the present invention, the capsule can be made of a substance chosen from a group comprising gelatine, chitosan, starch and/or starch derivatives, cellulose and/or cellulose derivatives or synthetic polymers as well as consisting intertwined upper and lower compartments.

In the case that the capsule used in the present invention is made of cellulose or its derivatives, the capsule material can be selected from, but not limited to, a group comprising hydroxypropyl cellulose, hydroxypropylmethyl cellulose, methyl cellulose, hydroxymethyl cellulose, hydroxyethyl cellulose.

In the case that the capsule used in the present invention is synthetic polymer, the capsule material can be selected from, but not limited to, a group comprising polyethylene, polyetheleneteraphtalate, polycarbonate or polypropylene.

In the case that the capsule material used in the present invention is gelatine, additional agents such as polyethylene glycol, sorbitol, glycerol, propylene glycol, polyethylene oxide - polypropylene oxide block copolymers and/or other polyalcohols or polyethers at different molecular weights can be added into it.

The dry powder drug pertaining to the present invention can also be stored in blister packs apart from reservoirs and capsules. Blister packs are comprised of orderly placed blisters each of which contains minimally one dose of the dry powder drug. Blister packs can be pierced or peeled to be opened according to the device design. However, peelable blister packs are preferred according to the present invention. When the device is actuated, the blister pack or one of the blisters in the pack is pierced or peeled and the drug in dry powder form is prepared for inhalation.

The cavity volume of the blisters pertaining to the present invention, which are placed side by side in an order and which provide to transmit and store the dry powder drug in blister pack, is in the range of 17-30 mm³ , preferably in the range of 18-23 mm^{3,} most preferably in the range of 19-21 mm^{3.}

The cavity volume of the blisters pertaining to the present invention, which provide to transmit and store the dry powder drug comprising tiotropium bromide having water content less than or equal to 2.5%, fluticasone propionate and sodium cromoglycate, is in the range of 17-30 mm³, preferably in the range of 18-23 mm^{3,} most preferably in the range of 19-21 mm³ and each blister cavity having the volume described above is filled up to 25-100 %, preferably up to 70-100 %, most preferably up to 90-100 % of said volume in order to meet the specified needs for an effective inhalation. The lid and the base sheets of said blister pack are closed very tightly by any suitable method to provide impermeability.

According to the present invention, the lid and the base sheet constituting the blister package consist of several layers. Polymeric layers, aluminum foil and preferably Aclar® fluoropoylmer film are among the layers that form the lid and the base sheet.

Aclar® fluoropolymer film is a polymeric film which is used in blister packs and provides excellent moisture barrier. This chemically inert polymeric film does not cause any change in the taste of the formulation when it is in contact with the dry powder formulation. In addition, it easily constitutes a layered structure with the other polymeric layers which are composed of various polymers. It is appropriate to be transacted with heat.

In order to decrease the gas and moisture permeability of the layer, preferably desiccant agents are added to the polymeric layers to preserve the stability of the dry powder formulation stored in blisters that are arranged in an order on blister strips. Silica gel, zeolite, alumina, bauxite, anhydrous calcium sulfate, activated carbon and clay which have the property of water absorption can be given as examples to desiccant agents.

As it is common to use aluminum in lid and base sheets of high protection blister packs, aluminum is used both in the lid and the base sheets of the blister pack of the present invention in order to provide high moisture and gas protection. These aluminum foils must be thick enough to provide the desired protection for the stability of the moisture sensitive dry powder formulation stored in the blister cavity. Due to this reason, the thickness of the aluminum foil that is used in the lid and the base sheets of the blister pack is chosen to be in the range of 10 to 40 µm, preferably of 15 to 30 µm.

The polymeric layers in the lid and the base sheets of the blister pack mentioned in the present invention are made from the same or different polymers. The thickness of these polymeric layers varies according to the type of the polymeric substance used and its properties. Therefore, the thickness of the polymeric layer varies in the range of 15-60 µm, preferably of 20-35 µm depending on the type of the polymer used.

The inside layer of the blister cavity of the said blister pack which is in contact with the dry powder formulation is a polymeric layer because of the fact that some of the dry powder formulation sticks onto the inside layer of the blister cavity due to the porous structure of aluminum foil and electrostatic forces, and hence causes uncontrolled dosing.

According to the present invention, the polymers used to form the polymeric layers are preferably selected from a group comprising thermo-plastic polymers such as polyethylene, polypropylene, polystyrene, polyolefin, polyamide, polyvinyl chloride, polyurethane or other synthetic polymers.

In addition, the blisters which constitute the blister pack pertaining to the present invention can be in any shape as long as they have the properties described above.

According to the present invention, tiotropium bromide with water content less than or equal to 2.5% can be in crystal form and/or amorphous form or a combination thereof.

According to the present invention, fluticasone propionate can be in the form of its solvates, hydrates enantiomers, diastereomers, racemates and/or in crystal form and/or amorphous form and/or a combination thereof.

According to the present invention, sodium cromoglycate can be in the form of its solvates, hydrates, and/or in crystal form and/or in amorphous form and/or a combination thereof.

According to the present invention, the amount of tiotropium bromide with water content less than or equal to 2.5% included in the drug formulation comprising tiotropium bromide with water content less than or equal to 2.5%, fluticasone propionate and sodium cromoglycate is in the range of 1-40 µg, preferably 1-30 µg per dose.

According to the present invention, the amount of fluticasone propionate included in the drug formulation comprising tiotropium bromide with water content less than or equal to 2.5%, fluticasone propionate and sodium cromoglycate is in the range of 1-600 µg, preferably 10-550 µg per dose.

According to the present invention, the amount of sodium cromoglycate included in the drug formulation comprising tiotropium bromide with water content less than or equal to 2.5%, fluticasone propionate and sodium cromoglycate is in the range of 1-50 mg, preferably 1-25 mg per dose.

The pharmaceutical composition mentioned in the present invention which contains tiotropium bromide with water content less than or equal to 2.5%, fluticasone propionate and sodium cromoglycate in dry powder form wherein;
- the mean particle size of tiotropium bromide with water content less than or equal to 2.5%, fluticasone propionate and sodium cromoglycate is not less than 1 µm and
- said active agents are present in the composition with the ratio of 1:2:40 to 1:50:2500 respectively and
- lactose which has a mean particle size less than 100 µm is used as carrier
can be used in the treatment of many respiratory diseases such as asthma, chronic obstructive pulmonary disorder (COPD) and allergic rhinitis. Accordingly, the respiratory diseases include, but not restricted to, allergic or non-allergic asthma at any phases, acute lung injury (ALI), acute respiratory distress syndrome (ARDS), exacerbation of airways hyperactivity, bronchiectasis, chronic obstructive pulmonary, airways or lung diseases (COPD, COAD or COLD) including emphysema and chronic bronchitis, pneumoconiosis, aluminosis, anthracosis, asbestosis, chalicosis, ptilosis, siderosis, silicosis, tabacosis and byssinosis. The treatment of said diseases may be prophylactic or symptomatic. In addition, the pharmaceutical composition pertaining to the present invention is used especially for the symptomatic treatment of asthma, allergic rhinitis and COPD.

A method for preparing the pharmaceutical composition according to the present invention comprises micronizing tiotropium bromide with water content less than or equal to 2.5%, fluticasone propionate and sodium cromoglycate, preferably by air jet mill, mixing the micronized tiotropium bromide with water content less than or equal to 2.5%, fluticasone propionate and sodium cromoglycate with lactose, then blending the composition to obtain a homogeneous dry powder mixture, and then filling the obtained dry powder mixture into capsules or blisters.

The pharmaceutical composition pertaining to the present invention can be explained in detail with, but not restricted to, the examples given below.

### EXAMPLES

### Example 1

A dry powder formulation which is appropriate for a gelatine capsule used in a capsule inhaler comprises 21 µg of tiotropium bromide with water content less than or equal to 2.5%, 250 µg of fluticasone propionate and 10 mg of sodium cromoglycate which are micronized by an air jet mill to obtain particles with a mean particle size not less than 1 µm and then the micronized particles are mixed with 5.75 mg of lactose as carrier which has a mean particle size less than 100 µm.

The active agent tiotropium bromide with water content less than or equal to 2.5% given in this example includes its all pharmaceutically acceptable amorphous and/or crystal forms thereof; fluticasone propionate includes its all pharmaceutically acceptable racemates, enantiomers or diastereomers, solvates, hydrates and/or amorphous and/or crystal forms and sodium cromoglycate includes its all pharmaceutically acceptable solvates, hydrates and/or amorphous and/or crystal forms. Lactose given in this example can be added in a higher or a lower amount. The capsule in this example is made of gelatin but it can optionally be made of chitosan, starch and/or starch derivatives, cellulose and/or cellulose derivatives or synthetic polymers.

The example 1 is repeated for each example given below providing that the amounts used in example 1 is replaced by the amounts in table of each example.

### Example 2

| Fluticasone Propionate | Tiotropium Bromide | Sodium Cromoglycate | Lactose |
|---|---|---|---|
| 250 µg | 11 µg | 10 mg | 5 mg |

### Example 3

| Fluticasone Propionate | Tiotropium Bromide | Sodium Cromoglycate | Lactose |
|---|---|---|---|
| 500 µg | 10.5 µg | 12 mg | 4 mg |

### Example 4

| Fluticasone Propionate | Tiotropium Bromide | Sodium Cromoglycate | Lactose |
|---|---|---|---|
| 50 µg | 15 µg | 11 mg | 3 mg |

### Example 5

| Fluticasone Propionate | Tiotropium Bromide | Sodium Cromoglycate | Lactose |
|---|---|---|---|
| 250 µg | 14 µg | 12 mg | 7 mg |

### Example 6

| Fluticasone Propionate | Tiotropium Bromide | Sodium Cromoglycate | Lactose |
|---|---|---|---|
| 250 µg | 21.5 µg | 13 mg | 3 mg |

### Example 7

| Fluticasone Propionate | Tiotropium Bromide | Sodium Cromoglycate | Lactose |
|---|---|---|---|
| 100 µg | 20 µg | 11 mg | 4.5 mg |

### Example 8

| Fluticasone Propionate | Tiotropium Bromide | Sodium Cromoglycate | Lactose |
|---|---|---|---|
| 100 µg | 12 µg | 15 mg | 3,5 mg |

### Example 9

| Fluticasone Propionate | Tiotropium Bromide | Sodium Cromoglycate | Lactose |
|---|---|---|---|
| 50 µg | 21 µg | 12.5 mg | 5.5 mg |

### Example 10

| Fluticasone Propionate | Tiotropium Bromide | Sodium Cromoglycate | Lactose |
|---|---|---|---|
| 100 µg | 12 µg | 10mg | 3.5mg |

### Example 11

| Fluticasone Propionate | Tiotropium Bromide | Sodium Cromoglycate | Lactose |
|---|---|---|---|
| 500 µg | 15 µg | 12 mg | 1.5 mg |

### Example 12

| Fluticasone Propionate | Tiotropium Bromide | Sodium Cromoglycate | Lactose |
|---|---|---|---|
| 50 µg | 12 µg | 12 mg | 7.5 mg |

### Example 13

So as to be used in a multiple dose inhaler, a dry powder drug formulation which is appropriate to be stored in blisters comprises 11.5 µg of tiotropium bromide with water content less than or equal to 2.5%, 500 µg of fluticasone propionate, 15 mg of sodium cromoglycate, that are micronized by air jet mill to obtain particles with a mean particle size not less than 1 µm, and 5.75 mg of lactose as carrier which has a mean particle size less than 100 µm.

The active substance tiotropium bromide with water content less than or equal to 2.5% given in this example includes its pharmaceutically acceptable amorphous and crystal forms thereof; fluticasone propionate includes its all pharmaceutically acceptable racemates, enantiomers or diastereomers, solvates, hydrates and/or amorphous and/or crystal forms and sodium cromoglycate includes its all pharmaceutically acceptable solvates, hydrates and/or amorphous and/or crystal forms. Lactose in this example can be added in a higher or a lower amount.

The example 1 is repeated for each example given below providing that the amounts used in example 1 is replaced by the amounts in table of each example.

### Example 14

| Fluticasone Propionate | Tiotropium Bromide | Sodium Cromoglycate | Lactose |
|---|---|---|---|
| 100 µg | 11.5 µg | 11.5 mg | 6 mg |

### Example 15

| Fluticasone Propionate | Tiotropium Bromide | Sodium Cromoglycate | Lactose |
|---|---|---|---|
| 250 µg | 12.5 µg | 10 mg | 5 mg |

### Example 16

| Fluticasone Propionate | Tiotropium Bromide | Sodium Cromoglycate | Lactose |
|---|---|---|---|
| 50 µg | 18 µg | 10.5 mg | 5.5 mg |

### Example 17

| Fluticasone Propionate | Tiotropium Bromide | Sodium Cromoglycate | Lactose |
|---|---|---|---|
| 250 µg | 11 µg | 14 mg | 4 mg |

### Example 18

| Fluticasone Propionate | Tiotropium Bromide | Sodium Cromoglycate | Lactose |
|---|---|---|---|
| 100 µg | 17.5 µg | 15 mg | 3.5 mg |

### Example 19

| Fluticasone Propionate | Tiotropium Bromide | Sodium Cromoglycate | Lactose |
|---|---|---|---|
| 250 µg | 15 µg | 14 mg | 6.5 mg |

### Example 20

| Fluticasone Propionate | Tiotropium Bromide | Sodium Cromoglycate | Lactose |
|---|---|---|---|
| 500 µg | 12 µg | 10 mg | 7.5 mg |

### Example 21

| Fluticasone Propionate | Tiotropium Bromide | Sodium Cromoglycate | Lactose |
|---|---|---|---|
| 100 µg | 20 µg | 15 mg | 4.5 mg |

### Example 22

| Fluticasone Propionate | Tiotropium Bromide | Sodium Cromoglycate | Lactose |
|---|---|---|---|
| 50 µg | 20.5 µg | 12.5 mg | 7.5 mg |

### Example 23

| Fluticasone Propionate | Tiotropium Bromide | Sodium Cromoglycate | Lactose |
|---|---|---|---|
| 500 µg | 13.5 µg | 18 mg | 3.5 mg |

### Example 24

| Fluticasone Propionate | Tiotropium Bromide | Sodium Cromoglycate | Lactose |
|---|---|---|---|
| 250 µg | 12 µg | 11 mg | 8.5 mg |

## Claims

1. A pharmaceutical composition for use in the symptomatic and/or prophylactic treatment of respiratory diseases comprising the simultaneous or sequential administration of tiotropium bromide with water content less than or equal to 2.5%, fluticasone propionate and sodium cromoglycate in dry powder form wherein
• the mean particle size of tiotropium bromide with water content less than or equal to 2.5%, fluticasone propionate and sodium cromoglycate is not less than 1 µm and
• said active agents are present in the composition with the ratio of 1:2:40 to 1:50:2500 respectively and
• lactose which is used as carrier has a mean particle size less than 100 µm.

2. The pharmaceutical composition for use according to claim 1, wherein the mean particle size of tiotropium bromide with water content less than or equal to 2.5% and fluticasone propionate is in the range of 1.5 to 4.5 µm.

3. The pharmaceutical composition for use according to claim 1, wherein the mean particle size of sodium cromoglycate is in the range of 1 to 300 µm.

4. The pharmaceutical composition for use according to claim 1, wherein the amount of fluticasone propionate and/or its solvates, hydrates enantiomers, diastereomers, racemates, crystal form and/or amorphous form and/or a combination thereof is in the range of 1-600 µg per dose.

5. The pharmaceutical composition for use according to claim 1, wherein said composition is used for simultaneous administration in the symptomatic and/or prophylactic treatment of respiratory diseases.

6. The pharmaceutical composition for use according to claim 1, wherein said pharmaceutical composition consists of tiotropium bromide with water content less than or equal to 2.5%, fluticasone propionate, sodium cromoglycate and lactose.

7. The pharmaceutical composition for use according to claim 1, wherein tiotropium bromide with water content less than or equal to 2.5%, fluticasone propionate, sodium cromoglycate are combined in a single dosage form.

8. The pharmaceutical composition for use according to claim 1, wherein the amount of sodium cromoglycate and/or its solvates, hydrates, and/or crystal form and/or amorphous form and/or a combination thereof is in the range of 1-50 mg per dose.

9. The pharmaceutical composition for use to claim 1, wherein the amount of tiotropium bromide with water content less than or equal to 2.5% and/or its crystal form and/or amorphous form or a combination thereof is in the range of 1-40 µg per dose.

10. The pharmaceutical composition for use according to claim 1, wherein lactose which has a mean particle size less than 100 µm is used as a mixture of particles having two different mean particle sizes.

11. The pharmaceutical composition for use according to claim 10, wherein lactose which has a mean particle size less than 100 µm is present as a mixture of particles having a mean particle size less than 10 µm and particles having a mean particle size in the range of 10 µm to 100 µm.

12. The pharmaceutical composition for use according to claim 11, wherein the weight ratio of lactose particles which have a mean particle size less than 10 µm (finer) to lactose particles which have a mean particle size in the range of 10 µm to 100 µm (coarser) is in the range of 1:1 to 1:25.

13. The pharmaceutical composition for use according to claim 11, wherein the ratio of lactose particles which have a mean particle size less than 10 µm (finer) to lactose particles which have a mean particle size in the range of 10 µm to 100 µm (coarser) is in the range of 1:1 to 1:10.

14. The pharmaceutical composition for use according to claim 11, wherein the ratio of lactose particles having a mean particle size less than 10 µm (finer) to lactose particles having a mean particle size in the range of 10 µm to 100 µm (coarser) is in the range of 1:1.5 to 1:5.

15. The pharmaceutical composition comprising tiotropium bromide with water content less than or equal to 2.5%, fluticasone propionate and sodium cromoglycate in dry powder form wherein
• the mean particle size of tiotropium bromide with water content less than or equal to 2.5%, fluticasone propionate and sodium cromoglycate is not less than 1 µm and
• said active agents are present in the composition with the ratio of 1:2:40 to 1:50:2500 respectively and
• lactose, which is used as carrier, has a mean particle size less than 100 µm is administered simultaneously or sequentially for use in treatment of respiratory diseases especially asthma, allergic rhinitis, chronic obstructive pulmonary disease (COPD).

## Patentansprüche

1. Eine pharmazeutische Zusammensetzung zur Verwendung bei der symptomatischen und / oder prophylaktischen Behandlung von Atemwegserkrankungen, die gleichzeitige oder sequentielle Anwendung von Tiotropiumbromid mit einem Wassergehalt weniger als oder gleich zu 2.5%, Fluticasonpropionat und Natriumcromoglycat in trockener Pulverform umfasst, wobei
• die mittlere Teilchengröße von Tiotropiumbromid mit einem Wassergehalt weniger als oder gleich zu 2.5%, Fluticasonpropionat und Natriumcromoglycat nicht weniger als 1 µm ist und
• die genannten Wirkstoffe in der Zusammensetzung mit dem Verhältnis von 1:2:40 bis 1:50:2500 jeweils vorhanden sind und
• Laktose, die als Träger verwendet wird, eine mittlere Teilchengröße weniger als 100 µm hat.

2. Pharmazeutische Zusammensetzung zur Verwendung nach Anspruch 1, wobei die mittlere Teilchengröße von Tiotropiumbromid mit einem Wassergehalt weniger als oder gleich zu 2.5% und Fluticasonpropionat im Bereich von 1,5 bis 4.5 µm liegt.

3. Pharmazeutische Zusammensetzung zur Verwendung nach Anspruch 1, wobei die mittlere Teilchengröße von Natriumcromoglycat im Bereich von 1 bis 300 µm liegt.

4. Pharmazeutische Zusammensetzung zur Verwendung nach Anspruch 1, wobei die Menge von Fluticasonpropionat und/oder seinen Solvaten, Hydraten, Enantiomeren, Diastereomeren, Racematen, Kristallformen und/oder amorphen Formen und/oder eine Kombination davon im Bereich von 1-600 µg pro Dosis liegt.

5. Pharmazeutische Zusammensetzung zur Verwendung nach Anspruch 1, wobei die Zusammensetzung für die gleichzeitige Anwendung in der symptomatischen und/oder prophylaktischen Behandlung von Atemwegserkrankungen eingesetzt wird.

6. Pharmazeutische Zusammensetzung zur Verwendung nach Anspruch 1, wobei die pharmazeutische Zusammensetzung Tiotropiumbromid mit einem Wassergehalt weniger als oder gleich zu 2.5%, Fluticasonpropionat, Natriumcromoglycat und Lactose umfasst.

7. Pharmazeutische Zusammensetzung zur Verwendung nach Anspruch 1, wobei Tiotropiumbromid mit einem Wassergehalt weniger als oder gleich zu 2.5%, Fluticasonpropionat und Natriumcromoglycat in einer einzigen Dosierungsform kombiniert sind.

8. Pharmazeutische Zusammensetzung zur Verwendung nach Anspruch 1, wobei die Menge von Natriumcromoglycat und/oder seinen Solvaten, Hydraten und/oder Kristallformen und/oder amorphen Formen und/oder eine Kombination davon im Bereich von 1-50 mg pro Dosis ist.

9. Pharmazeutische Zusammensetzung zur Verwendung nach Anspruch 1, wobei die Menge von Tiotropiumbromid mit einem Wassergehalt weniger als oder gleich zu 2.5% und/oder seinen Kristallformen und/oder amorphen Formen oder eine Kombination davon im Bereich von 1-40 µg pro Dosis ist.

10. Pharmazeutische Zusammensetzung zur Verwendung nach Anspruch 1, wobei Laktose, die eine mittlere Teilchengröße weniger als 100 µm hat, als eine Mischung von Teilchen verwendet wird, die zwei unterschiedlichen mittleren Teilchengröße aufweisen.

11. Pharmazeutische Zusammensetzung zur Verwendung nach Anspruch 10, wobei Laktose, die eine mittlereTeilchengröße weniger als 100 µm hat, als eine Mischung von Partikeln mit einer mittleren Teilchengröße weniger als 10 µm und Partikeln mit einer mittleren Teilchengröße im Bereich von 10 µm bis 100 µm vorhanden ist.

12. Pharmazeutische Zusammensetzung zur Verwendung nach Anspruch 11, wobei das Gewichtsverhältnis von Laktose-Teilchen, die eine mittlere Teilchengröße weniger als 10 µm (feinerer) haben, zu den Laktose-Teilchen, die eine mittlere Teilchengröße im Bereich von 10 µm bis 100 µm (gröber) haben, im Bereich von 1:1 bis 1:25 liegt.

13. Pharmazeutische Zusammensetzung zur Verwendung nach Anspruch 11, wobei das Verhältnis von Laktose-Teilchen, die eine mittlere Teilchengröße weniger als 10 µm (feiner) haben, zu den Laktose-Teilchen, die eine mittlere Teilchengröße im Bereich von 10 µm bis 100 µm (gröber) haben, im Bereich von 1:1 bis 1:10 liegt.

14. Pharmazeutische Zusammensetzung zur Verwendung nach Anspruch 11, wobei das Verhältnis von Laktose-Teilchen, die eine mittlere Teilchengröße weniger als 10 µm (feinerer) haben, zu den Laktose-Teilchen, die eine mittlere Teilchengröße im Bereich von 10 µm bis 100 µm (gröber) haben, in dem Bereich von 1:1,5 bis 1:5 liegt.

15. Pharmazeutische Zusammensetzung, enthaltend Tiotropiumbromid mit einem Wassergehalt weniger als oder gleich zu 2.5%, Fluticasonpropionat und Natriumcromoglycat in trockener Pulverform, wobei;
• die mittlere Teilchengröße von Tiotropiumbromid mit einem Wassergehalt weniger als oder gleich zu 2.5%, Fluticasonpropionat und Natriumcromoglycat nicht weniger als 1 µm ist und
• die genannten Wirkstoffe in der Zusammensetzung mit dem Verhältnis von 1:2:40 bis 1:50:2500 jeweils vorhanden sind und
• Laktose, die als Träger verwendet wird und eine mittlere Teilchengröße weniger als 100 µm hat, gleichzeitig oder nacheinander für die Verwendung bei der Behandlung von Atemwegserkrankungen insbesondere wie Asthma, allergische Rhinitis, chronischobstruktive Lungenerkrankung (COPD) eingesetzt wird.

## Revendications

1. La composition pharmaceutique utilisée pour le traitement symptomatique et/ou prophylectique de maladies respiratoires comprenant l'administration simultanée ou séquentielle de bromure de tiotropium avec une teneur en eau inférieure ou égale a 2.5%, propionate de fluticasone et coromoglycate de sodium sous forme de poudre sèche dans laquelle
• La dimension moyenne de particule de bromure de tiotropium avec une teneur en eau inférieure ou égale a 2.5 %, propionate de fluticasone et coromoglycate de sodium pas inférieure de 1 µm et,
• Lesdits agents actifs sont presents dans la composition avec une ratio de 1:2:40 à 1:50:2500 recpectivement.
• Lactose qui est utilisé oomme un support a une dimension moyenne de particule inférieure à 100 µm.

2. La composition pharmaceutique utilisée selon la revendication 1, la dimension moyenne de particule de bromure de tiotropium avec une teneur en eau inférieure ou égale a 2.5 %, propionate de fluticasone et coromoglycate de sodium inférieure de 1.5 à 4.5 µm.

3. La composition pharmaceutique utilisée selon la revendication 1, où la dimension moyenne de particule de coromoglycate de sodium dans l'intervalle de 1 à 300 µm.

4. La composition pharmaceutique utilisée selon la revendication 1 dans laquelle la quantité de propionate de fluticasone et/ou ses solvantes les hydrates enantiomères, diastéréomères, racemates, forme cristalline et/ou forme amorphe et/ou la combination de ceux-ci est dans l'intervalle de 1-600 µg par dosage.

5. Composition pharmaceutique pour utilisation selon la revendication 1, dans laquelle ladite composition est utilisée pour une administration simultanée dans le traitement symptomatique et / ou prophylactique de maladies respiratoires.

6. La composition pharmaceutique utilisée selon la revendication 1 dans laquelle ladite composition pharmacuetique consiste de bromure de tiotropium avec une teneur en eau inférieure ou égale a 2.5 %, propionate de fluticasone et coromoglycate de sodium et lactose.

7. La composition pharmaceutique utilisée selon la revendication 1 dans laquelle ladite composition pharmacuetique consiste de bromure de tiotropium avec une teneur en eau inférieure ou égale a 2.5 %, propionate de fluticasone et coromoglycate sont combinés soue une de dosage unique.

8. La composition pharmaceutique utilisée selon la revendication 1, dans laquelle la quantité de coromoglycate de sodium et/ou ses solvantes les hydrates , et/ou forme cristalline et/ou forme amorphe et/ou la combination de ceux-ci est dans l'intervalle de 1-50 mg par dosage.

9. La composition pharmaceutique utilisée selon la revendication 1 dans laquelle la quantité de bromure de tiotropium avec une teneur en eau inférieure ou égale à 2.5%, et/ou forme cristalline et/ou forme amorphe et/ou la combination de ceux-ci est dans l'intervalle de 1-40 µg par dosage.

10. La composition pharmaceutique utilisée selon la revendication 1 dans laquelle le lactose qui a une moyenne dimension de particule à l'inferieure de 100 µm, est utilisé comme un mélange de particules ayant deux differentes dimensions moyens de particules.

11. La composition pharmaceutique utilisée selon la revendication 10, dans laquelle le lactose qui a une moyenne dimension de particule à l'inferieure de 100 µm, est présent sous forme d'un mélange de particules ayant une dimension moyenne inféerieure à 10 µm et des particules ayant une dimension moyenne de particule dans l'intervalle de 10 µm à 100 µm.

12. La composition pharmaceutique utilisée selon la revendication 11, dans laquelle la ratio pondérale lactose a une dimension moyenne de particule inférieure à 10 µm (fine) au lactose qui a une dimension moyenne de particules dans l'intervalle de 10 µm à 100 µm (coarse) est dans l'intervalle de 1:1 à 1:25.

13. La composition pharmaceutique utilisée selon la revendication 11, dans laquelle la raitio de particules de lactose qui ont une dimension moyenne de particule inférieure à 10 µm (finer) de particules de lactose qui ont une dimension moyenne dans l'intervalle à 10 µm à 100 µm est dans l'intervalle de 1:1 à 1:10.

14. La composition pharmaceutique utilisée selon la revendication 11, dans laquelle la raitio de particules de lactose qui ont une dimension moyenne de particule inférieure à 10 µm (finer) de particules de lactose qui ont une dimension moyenne dans l'intervalle à 10 µm à 100 µm est dans l'intervalle de 1:1.5 à 1:5.

15. La composition pharmaceutique comprenant le bromure de tiotropium avec la teneur en eau inférieure ou égale à 2.5% propionate de fluticasone et coromoglycate de sodium sous forme de poudre sèche dans laquelle ,
• La dimension moyenne des particules de bromure de tiotropium avec une teneur en eau inférieure ou égale à 2.5%, propionate de fluticasone et coromoglycate de sodium ne sont pas a l'inférieure de 1 µm et
• Lesdits agents actifs sont présents dans la composition avec la ratio de 1:2:40 à 1:50:2500 respectivement et
• Le lactose qui est utilisé comme porteur a une dimension moyenne de particules inférieure à 100 µm est administré simultanément ou séquentiellement pour utilisation dans le traitement des maladies respiratoires, en particulier de l'asthme, la rhinite allergique, bronchopneumopathie chronique obstructive (BPCO).
